# EUROPEAN PATENT SPECIFICATION

(11) **EP 1 622 658 B1**
(45) Date of publication and mention of the grant of the patent: **25.08.2021**
(21) Application number: 04752282.6
(22) Date of filing: 13.05.2004
(51) Int. Cl.: A61M 16/00, A61M 25/00

(54) **APPARATUS FOR TREATING ASTHMA USING NEUROTOXIN**
GERÄT ZUR BEHANDLUNG VON ASTHMA MIT NEUROTOXIN
DISPOSITIF POUR TRAITER L'ASTHME AU MOYEN DE NEUROTOXINE

(30) Priority: 13.05.2003 US 437882
(43) Date of publication of application: 08.02.2006
(73) Proprietor: Nuvaira, Inc., Plymouth MN 55447 (US)
(72) Inventor: DEEM, Mark, E., Mountain View, California 94041 (US); GIFFORD, Hanson, S., Woodside, CA 94062 (US)
(74) Representative: Mathys & Squire
(86) International application number: PCT/US2004/015222
(87) International publication number: WO 2004/101028

(56) References cited:
- WO-A1-98/18391
- US-A- 5 766 605
- US-A- 5 766 605
- US-A- 5 964 223
- US-A- 5 964 223
- US-A1- 2002 111 620
- US-B1- 6 546 932
- US-B1- 6 546 932
- None

## Description

### Field of the Invention

The present invention relates to apparatus for treating asthma by controlled delivery of neurotoxin using a neurotoxin applicator assembly.

### Background of the Invention

The lung is made up of progressively smaller bronchial bifurcations stemming downward from the trachea. The trachea and proximal bronchi are lumens consisting of an outer layer of fascia surrounding a U-shaped inner cartilaginous layer, wherein the open portion of the U is spanned by smooth muscle. Inside the cartilaginous layer are a collagenous elastic layer and an innermost epithelial layer. Mucus secreting goblet cells and transport cilial cells are interspersed within these inner layers.

As the bronchi branch and get smaller, the cartilaginous layer changes from a U-shape to irregular and helical shapes. In addition, the smooth muscle layer becomes helical bands surrounding the entire circumference of the bronchi, the goblet cells gradually decrease in numbers and the ciliated cells get smaller and fewer in number. In the most distal bronchi, the outer cartilaginous layer disappears completely, the smooth muscle layer becomes the outermost layer and goblet cells and ciliated cells disappear completely.

Asthma is a complex disease of the bronchial tree, characterized by airway hyperresponsiveness to allergens, stress and environmental triggers. Environmental triggers include irritants such as pollutants and non-allergenic triggers such as exposure to cold air. Airway hyperresponsiveness results in acute narrowing of the entire bronchial tree reducing airflow through the lungs, compromising respiration and limiting gas exchange in the alveoli. The narrowing of the bronchial tree is a result of three basic characteristic physiologic responses: (1) smooth muscle contraction; (2) increased mucus production; and (3) edema caused by arterial dilatation and increased arterial permeability. The triggering mechanisms for these physiologic responses are part of the body's inflammatory response system.

Chronic uncontrolled asthma can result in structural changes to the bronchial wall itself. Smooth muscle hyperplasia results in thickening of the smooth muscle components of the bronchial wall. Thickening of the subepithelial collagen layer that lies between the airway epithelium and the smooth muscle layer results in progressive stiffening of the wall of the bronchi. Studies have shown that stiffening of the airway wall results in more profound narrowing of the airway for a given asthma attack. This is due to changes in the ability of the mucosal layer to fold in response to the smooth muscle layer contraction.

Recently, the controlled injection of neurotoxin has become a common procedure for controlling skeletal muscle spasms. A frequently used neurotoxin for this procedure is the botulinum toxin, serotype A, sold commercially by Allergan, Inc. as Botox™. Botulinum toxin blocks the release of neurotransmitter from the nerves that control the contraction of the target muscles. Many applications for botulinum toxin have been proposed and/or clinically tested, including cervical dystonia, cosmetic relief of frown lines and tremor associated with cerebral palsy. Recently, botulinum toxin has become the subject of clinical study for the relief of hyperhidrosis (profuse sweating) and hypersalivation. These studies indicate that botulinum toxin can be used to control the action of cholinergic parasympathetic nerves as well as large skeletal muscle groups. The recent findings open the possibility of using neurotoxins such as botulinum toxin to control some of the main mechanisms of airway narrowing in asthmatic attacks, specifically smooth muscle contraction and hypersecretion of mucus from the goblet cells. Additionally, there is evidence that some part of the inflammatory response of asthma is stimulated by the release of the neurotransmitters that botulinum toxin inhibits. This opens the possibility that botulinum toxin also may work to mitigate the inflammatory cycle itself.

The use of neurotoxin for the control of asthma is described in U.S. Patent No. 6,063, 768 to First, wherein asthma is included in a list of neurogenic inflammatory disorders that may be controlled through the action of neurotoxins such as botulinum toxin but does not describe the methods or devices used to do so. An earlier patent, U.S. Patent No. 5,766,605 to Sanders, et.al., describes the use of botulinum toxin to treat asthma and COPD. That patent also describes that botulinum toxin could be aerosolized and introduced into the lungs. Further mention of botulinum toxin in connection with asthma is provided in a press release dated February 7, 2003 by the University of Alberta in describing the work of Dr. Redwan Moqbel. The release mentions that Dr. Moqbel and others are researching the possible use of neurotoxins such as tetanus and botulinum toxin to prevent eosinophils from activating and starting the inflammatory cascade that results in an asthma attack.

While it may be possible to simply aerosolize neurotoxins for introduction into the lungs, introducing it into the patient through traditional inhalation means would expose the mouth, tongue, epiglottis, vocal cords, etc. to the actions of the neurotoxin, with obvious deleterious results. Much more controlled and direct application of the neurotoxin to the desired tissue is required for safe and effective therapy.

Accordingly, it would be desirable to provide apparatus that enables controlled delivery of a neurotoxin to target treatment areas within a patient's bronchial airways.

It also would be desirable to provide an apparatus permitting the controlled injection of neurotoxin into the bronchial wall of a patient.

It would further be desirable to provide a needle-less injection apparatus to eliminate potential complications related to the presence of needles within a patient's bronchial airways.

Additionally, it would be desirable to provide an apparatus permitting the application of neurotoxin onto a target treatment area within a patient's bronchial airways.

In addition, implantable drug eluting devices for treating various diseases also have been developed. Examples include drug eluting coronary stents such as those under clinical investigation and commercial sale from such companies as Boston Scientific and Cordis Corporation. Bioabsorbable devices for drug delivery have likewise been contemplated, such as those under development by Synecor, Inc. (recently acquired by Guidant), and by numerous university and hospital-based development efforts. Examples of such efforts are provided in Blindt, et al., Development of a New Biodegradable Intravascular Polymer Stent with Simultaneous Incorporation of Bioactive Substances, Int'l J. Artif. Organs, 22(12):843-853 (1998) and Tsuji, et al., Biodegradable Stents as a Platform to Drug Loading, Int'l J. Cardio. Interv., 13-16 (2003).

Other uses for biodegradable stents include restoring patency of the urethra following treatment of prostatic hyperplasia and maintaining the patency of bronchial airways after impingement by lung tumors. Biodegradable and non-biodegradable implants for the controlled release of neurotoxin for the treatment of various diseases is described in U.S. Patent Nos. 6,306,423, 6,506,399 and 6,383,509. Further, U.S. Patent No. 5,766,605 describes the use of neurotoxin to treat autonomic nerve dysfunction, including specific claims involving treating asthma and chronic obstructive pulmonary disease (COPD). Additionally, U.S. Patent Publication No. 2002/0082197 describes the use of neurotoxin for treating mucus secreting disease, such as asthma and COPD, as does WO 00/10598.

It further would be desirable to provide implantable apparatus that provides a sustained or periodic dosage of asthma-fighting drugs to a target treatment area within a patient's bronchial airways.

US 5,964,223 describes a method and apparatus for delivering a medicine to a patient via the patient's respiratory system. A nebulization catheter is positioned in the patient's respiratory system so that a distal end of the nebulization catheter is in the respiratory system and a proximal end is outside the body. The nebulization catheter conveys medicine liquid form to the distal end at which location the medicine is nebulized by a pressurised gas or other nebulizing mechanisms.

US 5,766,605 describes a method for the control of autonomic nerve function in a mammal which method comprises administering a therapeutically effective amount of botulinum toxin to the mammal.

WO 98/18391 describes an apparatus for delivering a medicament into an injection site in or beyond a vessel wall or vessel obstruction including an intraluminal catheter containing: at least one puncturing element; an ultrasound emitter located in the proximity of the extendable puncturing element(s); and an introducer element that introduces the medicament into an injection site that is in or beyond the vessel wall, but within a portion of the vessel wall that has been penetrated by the puncturing element.

### Summary of the Disclosure

In view of the foregoing, it is an object of the present disclosure to provide apparatus that enables the controlled delivery of a neurotoxin to target treatment areas within a patient's bronchial airways.

It is a further object of the present disclosure to provide an apparatus permitting the controlled injection of neurotoxin into the bronchial wall of a patient.

It is an additional object of the present disclosure to provide a needle-less injection apparatus to eliminate potential complications related to the presence of needles within a patient's bronchial airways.

It is another object of the present disclosure to provide an apparatus permitting the application of neurotoxin onto a target treatment area within a patient's bronchial airways.

It still further is an object of this disclosure to provide implantable apparatus that provides a sustained or periodic dosage of asthma-fighting drugs to a target treatment area within a patient's bronchial airways.

These and other objects
are accomplished by providing an intrabronchial neurotoxin delivery system for controlled delivery of neurotoxin to a target treatment area within a patient's bronchial airways to lessen the effects of asthma. The introduction of neurotoxin into the bronchial airways disables the hyperresponsive smooth muscle layer and controls the hypersecretion of mucus.

The present invention is set out in the appended claims. Described herein is a intrabronchial neurotoxin delivery system comprising neurotoxin applicator assembly. The distal end of the neurotoxin applicator assembly may comprise a needle tip, a rotating needle assembly, a needle-less injection assembly, a nebulizer, or a micro-porous tip. This and all following applicator embodiments may be designed to work through or in concert with a standard bronchoscope, or may be incorporated into a stand-alone system for accessing and treating the airways.

In accordance with this aspect
the neurotoxin applicator assembly comprises an elongated shaft having a lumen in fluid communication with a source of liquid neurotoxin. The distal end of the shaft includes one or more needles that are preformed to contract radially when disposed within a lumen of the bronchoscope, but may be extended to penetrate and inject small doses of neurotoxin into the bronchial wall of a patient.

Alternatively, the distal end of the neurotoxin applicator assembly may carry a rotating needle assembly including plural needles disposed along the circumference of a wheel, so that the wheel may be rolled across a target treatment area. Optionally, the rotating needle assembly may include a fender to protect a portion of the bronchial wall substantially opposite the target treatment area.

In another alternative embodiment, the distal end of the neurotoxin applicator assembly may comprise a needle-less injection assembly that may be used to inject neurotoxin into the bronchial wall without needle penetration. An inflatable balloon optionally may be provided to help position a port of the needle-less injection assembly adjacent to the target treatment area.

In a further alternative embodiment, the distal end of the neurotoxin applicator assembly comprises a nebulizer assembly having an atomizer that converts the liquid neurotoxin into a fine spray or mist that is directed onto the target treatment area. The particle size of the mist can be controlled using injection pressure or atomizer head design to access specific portions of the lung adjacent to or downstream of the treatment device. An inflatable balloon optionally may be provided to facilitate positioning the atomizer adjacent the target treatment area, and further serves to isolate the lung segment downstream of the device to prevent reflux of the mist into undesired portions of the airway. In addition, lumens optionally may be disposed between the balloon and atomizer to provide a ventilation system that allows pressure control of the treatment area to prevent over-inflation of the lung, mixing of the atomized fluid, and evacuation of remaining mist at termination of therapy, prior to balloon deflation.

In a still further embodiment, the distal end of the neurotoxin applicator assembly may comprises an articulable region of the shaft including a micro-porous tip that mediates flow of asthma-fighting solution through the lumen and out of the distal tip.

Described herein is a intrabronchial neurotoxin delivery system comprising a drug-eluting bioabsorbable stent comprising one or more layers of asthma-fighting drugs.

### Summary of the Invention

The invention is defined in claim 1 and further defined in the appended claims. Brief Description of the Drawings

The above and other objects and advantages will be apparent upon consideration of the following detailed description, taken in conjunction with the accompanying drawings, in which like reference characters refer to like parts throughout, and in which:
FIG. 1 is a side view of an intrabronchial neurotoxin delivery system embodying the claimed invention ;
FIG. 2 is a perspective view of an illustrative embodiment of a neurotoxin applicator assembly embodying the claimed invention;
FIGS. 3A and 3B are cross-sectional views of the neurotoxin applicator assembly of FIG. 2 in retracted and extended positions, respectively;
FIG. 4 is a perspective view of an alternative embodiment of a neurotoxin applicator assembly;
FIGS. 5A and 5B are partial cross-sectional views of the neurotoxin applicator assembly of FIG. 4 in retracted and extended positions, respectively;
FIG. 6 is a perspective view of another alternative embodiment of a neurotoxin applicator assembly;
FIG. 7A and 7B are partial cross-sectional views of the neurotoxin applicator assembly of FIG. 6 in retracted and extended positions, respectively;
FIG. 8 is a perspective view of a yet further alternative embodiment of a neurotoxin applicator assembly;
FIGS. 9A and 9B are partial cross-sectional views of the neurotoxin applicator assembly of FIG. 8 in retracted and extended positions, respectively;
FIG. 10 is a side view of a distal end of a neurotoxin applicator assembly for topically applying an asthma-fighting solution to a patient's bronchi;
FIGS. 11A-11C are side views of the applicator of FIG. 10 depicting the curved distal segment in different configurations;
FIG. 12 is a perspective view of a syringe that is employed to provide pressurization at the proximal end of the applicator of FIG. 10;
FIG. 13 is a perspective view of an alternative syringe that is employed to provide pressurization at the proximal end of the applicator of FIG. 10;
FIG. 14 is a side view of a drug-eluting bioabsorbable stent ;
FIG. 15 is a side view of another drug-eluting bioabsorbable stent .;
FIG. 16 is a cross-sectional view of the drug-eluting bioabsorbable stent of FIG. 14;
FIG. 17 is a perspective view depicting the delivery of drug-eluting bioabsorbable stents within a patient's bronchi;
FIG. 18 is a perspective view depicting the delivery of alternative drug-eluting bioabsorbable stents within a patient's bronchi; and
FIG. 19 is a perspective view depicting the delivery of further alternative drug-eluting bioabsorbable stents within a patient's bronchi.

### Detailed Description

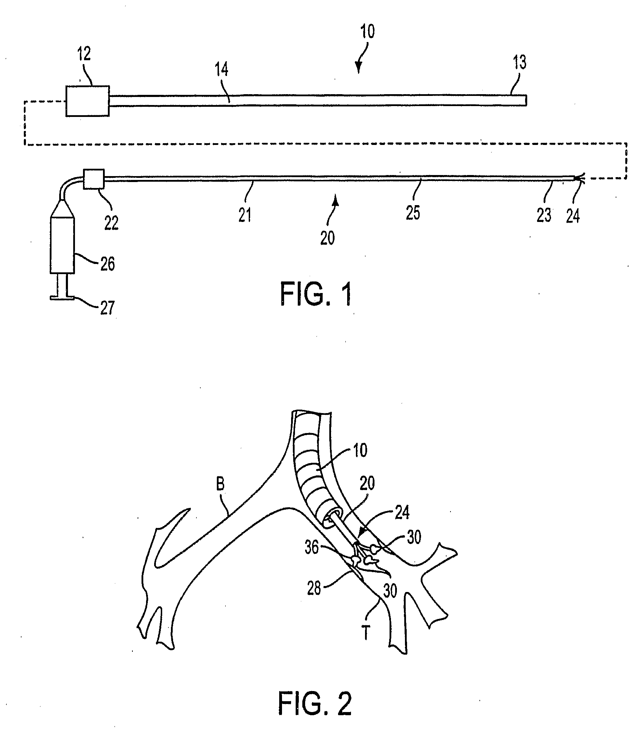

Referring to FIG. 1, apparatus for controlled delivery of neurotoxin to a target treatment area within a patient's bronchial airways to lessen the effects of asthma is described. Preferably, the apparatus comprises bronchoscope 10 and neurotoxin applicator assembly 20. Bronchoscope 10 has proximal end 12, distal end 13, and lumen 14. As is conventional, bronchoscope 10 also includes a light source for illuminating the interior of a patient's lung and optics, such as a miniature camera, that enables the physician to view the interior of the patient's lung. Alternatively, bronchoscope 10 may omit the light source and optics, and instead comprise an outer sheath. In this latter case, device 10 and neurotoxin applicator 20 would be observed using a separate conventional bronchoscope or through a light source and optics that are built into the neurotoxin applicator 20.

Neurotoxin applicator assembly 20, of which various illustrative embodiments are described hereinbelow, enables the physician to selectively administer controlled doses of neurotoxin to or within selected treatment sites in the patient's lung. More specifically, neurotoxin applicator assembly 20 may be selectively advanced through lumen 14 of bronchoscope 10 to deliver a neurotoxin, such as botulinum toxin, serotype A, to a target treatment area.

Neurotoxin applicator assembly 20 includes shaft 21 coupled to at its proximal end to handle 22, distal end 23 having neurotoxin applicator 24, and lumen 25. Lumen 25 provides fluid communication between proximal end and handle 22 and applicator 24. Syringe 26 having plunger 27 is coupled to a port on proximal end 22. Syringe 26 is filled with neurotoxin in liquid form, and applies the neurotoxin to applicator 24 via lumen 25 when plunger 27 is actuated.

Handle 22 enables the physician to extend and retract applicator 24 from within lumen 14 of bronchoscope 10, and to manipulate distal end 23 of neurotoxin applicator assembly 20 under direct visual observation using the optics of bronchoscope 10. The neurotoxin applicator assembly preferably remains retracted within lumen 14 of the bronchoscope during insertion of the catheter into the patient's bronchial airways, and is deployed once the applicator is in a desired position. Alternatively, applicator 20 may be housed inside of a retaining sheath, and both units can be advanced through lumen 14 together.

Referring now to FIGS. 2-3, a first illustrative embodiment of applicator 24 of neurotoxin applicator assembly 20
is described. Applicator 24 comprises needle assembly 28 having at least one needle 30 with lumen 31 in fluid communication with lumen 25. The needles are configured to penetrate the airway epithelium and directly inject small amounts of neurotoxin from the syringe into the collagenous and smooth muscle layers of bronchial wall **B.**

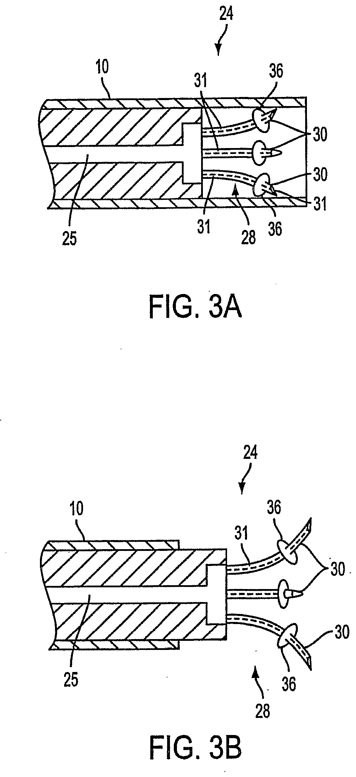

In FIG. 3A, needle assembly 28 is depicted retracted with lumen 14 of bronchoscope 10. Alternatively, device 10 may comprise an outer sheath that is dimensioned to be slidably accept neurotoxin applicator assembly 20, and which is selectively retractable to expose needle assembly 28. Again, in the case where device 10 is an outer sheath, visualization could be achieved through a separate bronchoscope or through built-in optics. In a further embodiment, a retaining sheath housed within lumen 14 and covering applicator 20 is selectably retractable to expose needle assembly 28. As depicted in FIG. 3B, needles 30 comprise a material capable of retaining a preformed shape, such as nickel-titanium, and are preformed to deflect radially outward when extended beyond distal end 13 of bronchoscope 10 (or the distal end of the outer sheath, if present). Each needle 30 includes hilt 36 disposed a pre-selected distance from the distal end of the needle to control the depth of penetration of the needle tip into the bronchial wall.

When needle assembly 30 is deployed, as illustrated in FIGS. 2 and 3B, needles 30 penetrate target treatment area T of bronchial wall B so that neurotoxin may be injected in the bronchial wall. Syringe 26 may include graduations that enable the physician to inject a pre-determined amount of neurotoxin at each target treatment area.

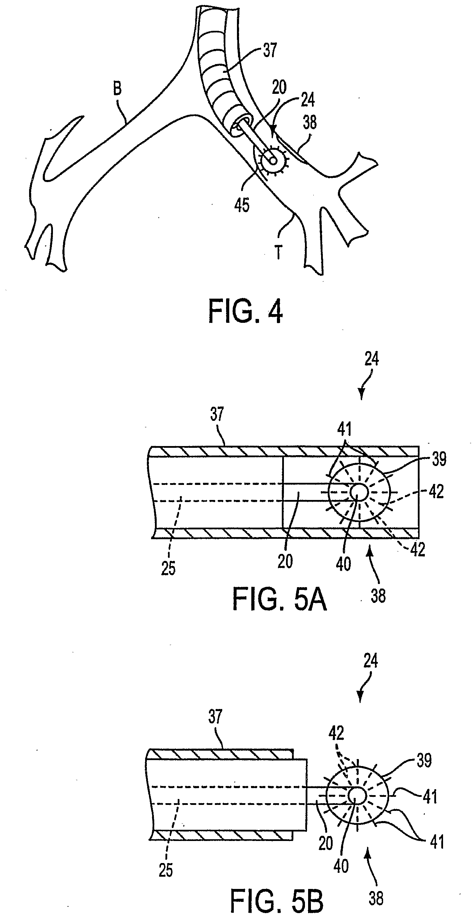

Referring now to FIGS. 4 and 5, an alternative embodiment of applicator 24 of neurotoxin applicator assembly 20 is described. Applicator 24 in this embodiment comprises rotating needle assembly 38, including wheel 39 mounted to rotate about hub 40. While wheel 39 illustratively is round, it alternatively may comprise a ellipse or hexagon or other polygonal shape. Plurality of needles 41 is disposed around the circumference of the wheel, each needle 41 having lumen 42 in fluid communication with lumen 25 via a passageway in hub 40. Optional fender 45 protects a portion of the bronchial wall substantially opposite the target treatment area.

In FIG. 5A, rotating needle assembly 38 is shown retracted within outer sheath 37. Outer sheath 37 is dimensioned to fit within lumen 14 of bronchoscope 10, and may be selectively retracted to expose rotating needle assembly 38. Alternatively, rotating needle assembly 38 extends through lumen 14 and past the tip of bronchoscope 10. In this embodiment, the wheel is covered by a retractable protection sheath which covers the wheel during insertion of the system. In FIGS. 4 and 5B, rotating needle assembly 38 is shown in the extended position. When so deployed, wheel 39 may be rolled across target treatment area **T,** so that as the wheel rotates needles 41 alternately penetrate and inject neurotoxin into bronchial wall **B.**

Suitable needles materials for needle assembly 28 of FIGS. 2-3 and rotating needle assembly 38 of FIGS. 4-5 include shape memory alloys such as nickel titanium alloys and spring tempered stainless steel alloys. Advantageously, either needle assembly permits direct injection of neurotoxin into the bronchial wall. This prevents the cilial transport system from trapping the neurotoxin and transporting it to other regions of the respiratory system, e.g., the oropharynx, where potentially unintended targets may be exposed to the neurotoxin, and prevents accidental exhalation of aerosolized neurotoxin.

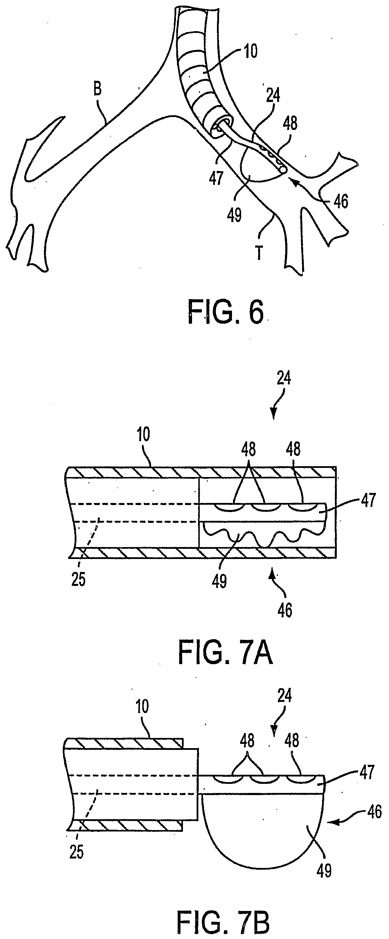

Referring now to FIGS. 6 and 7, another alternative embodiment of applicator 24 of the neurotoxin applicator assembly is described. Applicator 24 of FIGS. 6-7 comprises a needle-less injection assembly 46, which uses pressurized injection to deliver neurotoxin from the proximal controller to target treatment area **T.** Advantageously, the needle-less injection assembly allows controlled introduction of neurotoxin across the airway epithelium without the potential complications of introducing needles proximate to the delicate bronchial tissues, and may allow a lower profile system.

Needle-less injection assembly 46 comprises shaft 47 including at least one port 48 in fluid communication with lumen 25. Inflatable balloon 49 optionally may be coupled to shaft 47, and used to position the shaft adjacent target treatment area **T.** Balloon 49 is inflated with a fluid introduced through a lumen of shaft 47. When the shaft is aligned with the target treatment area, pulses of pressurized gas may be employed to inject predetermined amounts of neurotoxin across the airway wall and into the collagenous and smooth muscle layers.

In FIG. 7A, needle-less injection assembly 46, with balloon 49 deflated, is depicted housed within the lumen 14 of bronchoscope 10 (or a separate outer sheath). FIGS. 6 and 7B depict needle-less injection assembly 46 with balloon 49 inflated to place ports 48 in apposition to target treatment area **T.** Once the physician has confirmed placement of needle-less injection assembly 46, e.g., by visualization using the optics of bronchoscope 10, x-ray, fluoroscopy or other suitable means, a controller attached to the proximal end of neurotoxin applicator assembly 20 (instead of syringe 26), may be activated to deliver the desired doses of neurotoxin to the bronchial wall. As an alternative to the balloon 49, the assembly may have 2 or more needle-less injectors arranged to position against opposite walls of the bronchial passage. For instance, they might be spring loaded to expand the sections away from the midline and contact the bronchial wall. As a further alternative, the shaft of the assembly may be pre-curved or actively curved with an activation mechanism to urge the injector against the wall of the bronchial passage. Yet another alternative includes lower-pressure injection of neurotoxin through port(s) 48, creating a weeping-balloon applicator. In this case, transport of neurotoxin across the epithelial surface is achieved by cellular endocytosis of the neurotoxin.

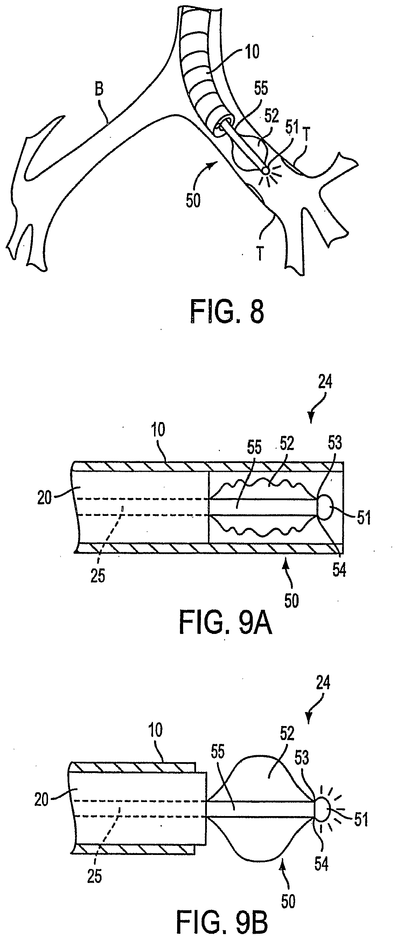

With respect to FIGS. 8 and 9, a yet further alternative embodiment of applicator 24 of the neurotoxin applicator assembly constructed
is described. Applicator 24 comprises nebulizer assembly 50 having shaft 55 with atomizer 51 disposed at its distal end and in fluid communication with central lumen 25. Atomizer 51 converts the liquid neurotoxin from the syringe into a fine spray or mist. Particle size of the mist can be controlled through nebulizer head design or by varying injection pressure in order to control the depth of penetration of the mist into the target segment.

Nebulizer assembly 50 may also include optional inflatable balloon 52 disposed on shaft 55 proximal of atomizer 51. Selective inflation of balloon 52 allows positioning of atomizer 51 so that aerosolized neurotoxin may be directly sprayed onto target treatment area T. Balloon 52 also acts to isolate the treatment area from the rest of the lung, preventing reflux of mist into unintended areas. As for the embodiment of FIGS. 6-7, balloon 52 may be inflated using a fluid introduced through an auxiliary lumen in shaft 55.

In FIG. 9A, the nebulizer assembly, including deflated balloon 52, is disposed within lumen 14 of bronchoscope 10, or alternatively, in an outer sheath that is slidably received in lumen 14. Alternatively, the nebulizer assembly may be inserted within a separate delivery sheath, with the bronchoscope inserted separately or with the optics and/or steering mechanisms built into the nebulizer assembly. In FIGS. 8 and 9B, nebulizer assembly 50 is depicted deployed from lumen 14 (or the outer sheath, if present), with balloon 52 inflated. Advantageously, nebulizer assembly 50 can be dimensioned to access very small bronchial passageways, and also may be used to deliver neurotoxin to upstream regions of the lung.

Still referring to FIGS. 9A and 9B, shaft 55 optionally also may include an additional auxiliary lumen or lumens coupled to inlet port 53 and outlet port 54 disposed between the balloon and the atomizer. Inlet port 53 allows the introduction of gas (such as fresh air) near the target treatment area, while outlet port 54 allows air or gas mixed with atomized neurotoxin to be removed. Inlet and outlet ports 53 and 54 therefore provide a ventilation system that shields tissue adjacent and proximal to target treatment area **T** from being inadvertently exposed to the atomized neurotoxin. Inlet and outlet ports 53 and 54 further serve to either actively inflate and deflate the isolated segment, or simply to normalize pressure within the lung near the target treatment area. A control unit may be connected to the proximal outlets of ports 53 & 54 to control the introduction and removal of gases from the lung without allowing escape of atomized neurotoxin to the environment or patient.

According to some embodiments, the nebulizer assembly may be built into a single, disposable fiberoptic unit having a unidirectional tip-deflection element having an integral isolation balloon and aerosolizing tip. A neurotoxin containing canister can also be provided integral with the nebulizer assembly. Advantageously, such an assembly permits a much lower profile, user-friendly system suitable for trans-nasal introduction past the vocal cords and into the lung.

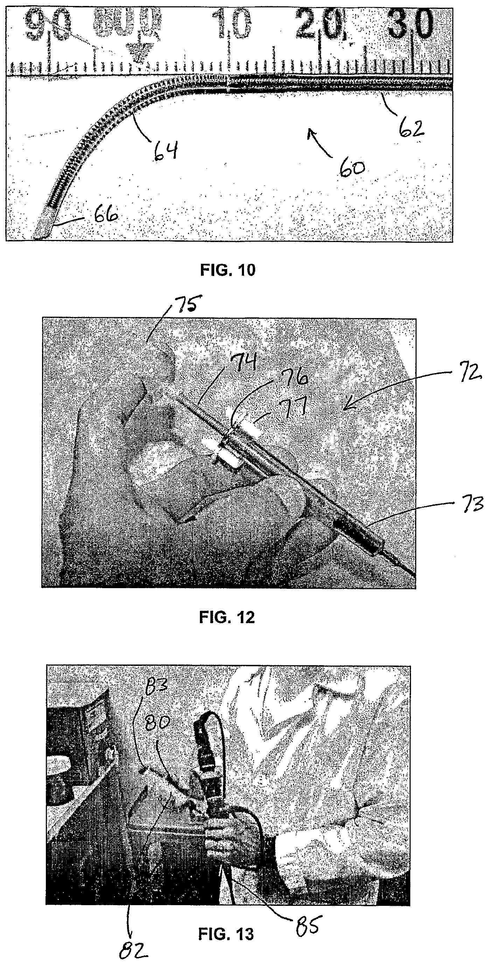

With respect to FIGS. 10-13, another alternative embodiment of a neurotoxin applicator
is described. In this embodiment, neurotoxins or other therapeutic drugs are delivered to the patient's bronchial tree via sub-selective topical application of toxin to the bronchial lining. The applicator assembly may be inserted through the working channel of a bronchoscope (as in FIG. 1) and used to paint a solution containing neurotoxins onto the epithelial surface of the airways of the lung.

Referring now to FIG. 10, neurotoxin applicator assembly 60 comprises proximal shaft 62, springreinforced distal segment 64, and beveled distal tip 66 comprising a micro-porous filter material that functions as a topical applicator for the asthma-fighting solution. Distal segment 64 preferably is bendable into an infinite number of configurations having various radii of curvature. Additionally, torsional rotation of distal tip 66 is provided in response to torque applied to proximal shaft 62. In the illustrated embodiment, neurotoxin applicator assembly 60 comprises a wire-braid reinforced tube that provides desirable characteristics of high burst strength and good torque response, and may be formed of a nickel-titanium alloy.

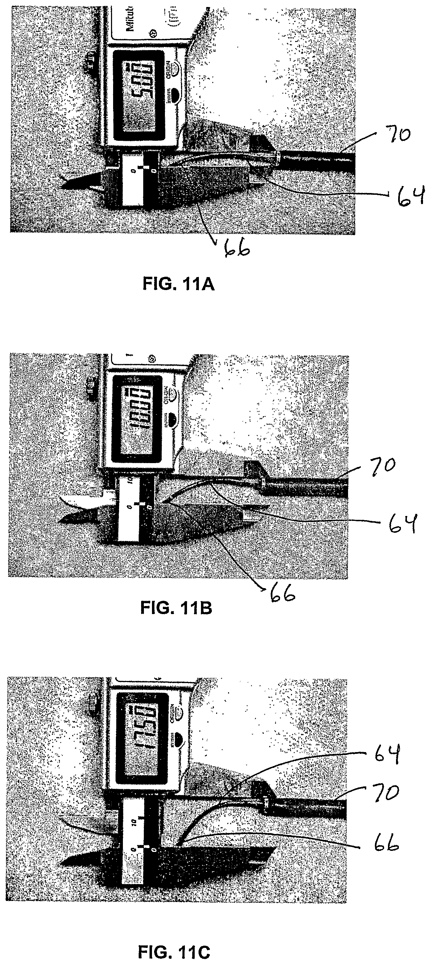

Referring to FIGS. 11A-11C, curved distal segment 64 of neurotoxin applicator assembly 60 extends out of the tip of bronchoscope 70. Neurotoxin applicator assembly 60 preferably may be rotated with respect to and in conjunction with bronchoscope 70. The variable curve of distal segment 64 and the beveled termination of distal tip 66 provide intimate contact of distal tip 66 against bronchial walls having different diameters. The beveled distal tip 66 allows for easy introduction through the working channel of the bronchoscope 70. Distal segment 64 preferably is spring-biased into a curved configuration to permit self-adjustment of the device to a wide range of inner bronchial diameters. FIGS. 11 demonstrate that the curved distal segment will remain in contact with the walls of very small bronchi having a diameter of 5 mm or less (FIG. 11A), average bronchi having a diameter of 5 mm to 15 mm (FIG. 11B), as well as very large bronchi having a diameter of 15 mm or more (FIG. 11C).

In operation, the asthma-fighting solution is induced to flow through a central lumen running through neurotoxin applicator assembly 60. According to some embodiments, the rotation of distal tip 66 using torque applied at the proximal end of proximal shaft 62, in addition to proximal end pressure, is used to control the flow of solution through distal tip 66. This flow control aspect, in combination with the variable curve of distal segment 64, allows a rotational "painting" onto the walls of the bronchi.

Referring to FIG. 12, syringe 72 is employed to provide pressurization at the proximal end of neurotoxin applicator assembly 60 for controlling the flow of asthma-fighting solution through the central lumen and distal tip of the device. Syringe 72 comprises syringe barrel 73 and threaded plunger 74, which terminates in finger slot 75. A proximal end of syringe barrel 73 terminates in an outwardly-projecting rim 76, whereas a distal end of syringe barrel 73 is fixedly attached to the proximal end of torque-responsive neurotoxin applicator assembly 60. Syringe 73 preferably further comprises a threaded half-collar 77 adapted to slide into place over syringe barrel 73 and threaded plunger 74. Half-collar 77 preferably comprises internal threads corresponding to the threads on threaded plunger 74. Moreover, half-collar 77 preferably further comprises a recess that engages rim 76. When slid into place over syringe 72, threaded half-collar 77 locks the threaded plunger in place with respect to the barrel.

When collar 77 is in place, rotation of syringe barrel 73 (e.g., in a clockwise direction) results in a controlled deflection of plunger 74 relative to barrel 73. The deflection of plunger 74 increases the pressure within the central lumen of neurotoxin applicator assembly 60, thereby causing a controlled amount of solution to be dispensed from distal tip 66. By altering the pitch of the thread on the plunger and half-collar, the volume of fluid dispensed per revolution may be varied. Since syringe barrel 73 is fixedly attached to torque-responsive neurotoxin applicator assembly 60, rotation of syringe barrel 73 results in corresponding rotation of distal tip 66. The half-collar may be removed from the assembly to allow conventional syringe operation for rapid filling or emptying of syringe 72.

Referring to FIG. 13, standard syringe 80 is attached to the proximal end of torque-responsive neurotoxin applicator assembly 60, which is disposed within bronchoscope 85. Syringe comprises syringe barrel 82 and non-threaded plunger 83. In this embodiment, a collar is not provided, and flow control is provided by manually modulating the force applied to plunger 83. The force applied to plunger 83, in addition to the rotation of syringe 80 (which causes corresponding rotation of neurotoxin applicator assembly 60) dispenses the asthma-fighting solution onto the patient's bronchial walls.

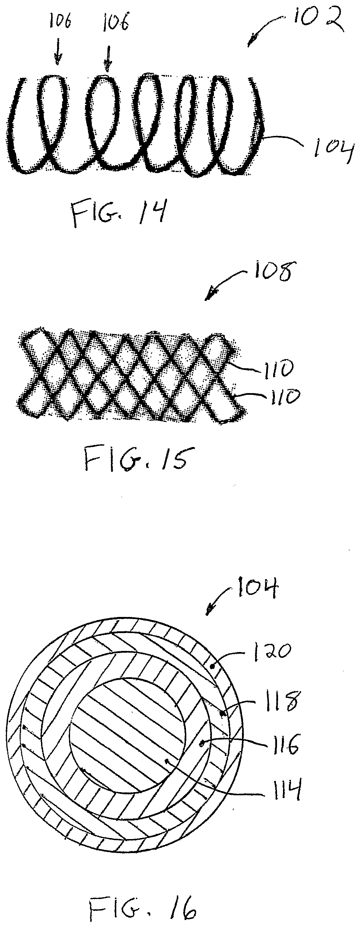

Implantable devices and methods for treating asthma are described with respect to FIGS. 14-19. The bioabsorbable implants are employed to controllably introduce one or more neurotoxins into the walls of a patient's bronchial tree, which control asthma by disabling the hyperresponsive smooth muscle layer and controlling hypersecretion of mucus.

More specifically, delivery of neurotoxins or other therapeutic drugs to the bronchial tree is achieved through drug elution from bioabsorbable implants placed within the bronchial passages. The implants preferably are bioabsorbable, but alternatively may comprise permanent devices with refillable reservoirs as well as elution devices that may be removed and replaced at periodic intervals, e.g., when drug supplies are determined be depleted.

A system for treating asthma comprises one or more drug-eluting bioabsorbable stents. Referring to FIG. 14, stent 102 comprises a single wire 104 including a plurality of turns 106. According to some embodiments, the stent system comprises one or more discrete-length coiled stents. Alternatively, the stent system comprises one or more custom-length coiled stents, which are deployed and cut to length at the end point of delivery by a cutting mechanism that is part of the stent delivery system. Bioabsorbable stent 102 preferably is loaded with neurotoxin and/or other asthma-fighting drugs. Gradual elution of the neurotoxin and/or other asthma-fighting drugs treats the underlying inflammatory disease that causes the asthmatic response. Advantageously, stent 102 also lends mechanical reinforcement to the patient's bronchi.

Referring to FIG. 15, alternative drug-eluting bioabsorbable stent 108 is shown, and comprises a plurality of wires 110 that are intertwined to form a slotted or knitted tube. As will be appreciated by those of skill in the art, many other known stent designs may be used to deliver the asthma-fighting drugs.
Suitable materials for stents 102, 108 include PEG, PLLA, collagen and copolymers of these materials. Appropriate drugs include the array of steroids and bronchodilators that are currently used systemically to treat asthmatic conditions. Examples of other suitable asthma-fighting drugs include corticosteroid and albuterol.

Additionally, a neurotoxin composition such as botulinum toxin or tetanus may be delivered using the stent. According to some embodiments, stents 102 encapsulate the neurotoxin, releasing it gradually onto the bronchial wall, thereby greatly extending the effective working life of the neurotoxin past its usual 3+ month active lifespan.

Since the effect of neurotoxin on the smooth muscle and inflammatory responses related to asthma are not permanent, it is desirable to provide doses of neurotoxin that are automatically delivered at predetermined intervals. FIG. 16 is a cross-sectional view of stent wire 104 of drug-eluting bioabsorbable stent 102. Wire 110 forming drug-eluting bioabsorbable stent 108 may comprises a substantially similar cross-section.

In the illustrated embodiment, stent wire comprises alternating layers of drug-loaded and non-drug-loaded resorbable materials. More particularly stent wire 104 comprises inner drug-eluting layer 114 that is substantially circular in cross-section and barrier layer 116 disposed around the perimeter of inner layer 114. Stent wire 104 further comprises outer drug-eluting layer 118 surrounding barrier layer 116, and protective outer coating 120 surrounding outer layer 118. In this manner, the drug is loaded into the implant in discrete dosing layers 114, 118 and delivered at predetermined intervals.

After the drug-eluting stent is implanted within a patient's bronchial airways, protective outer coating 20 dissolves, thereby exposing outer drug-eluting layer 118. The outer layer is slowly absorbed, thus providing a first dose of asthma-fighting drugs to a target treatment area within the patient's bronchi. After outer layer 118 is absorbed, barrier layer 116 dissolves within the bronchi over a predetermined amount of time. Then, inner layer 114 is slowly absorbed, thereby providing a second dose of asthma-fighting drugs to the target treatment area. Of course, as would be understood by those of ordinary skill in the art, additional drug-eluting layers (and barrier layers) may be employed to deliver additional doses of asthma-fighting drugs at predetermined intervals.
In this manner, the first dose is administered shortly after implantation, and then subsequent doses are released at predetermined intervals, for example every 3 months.

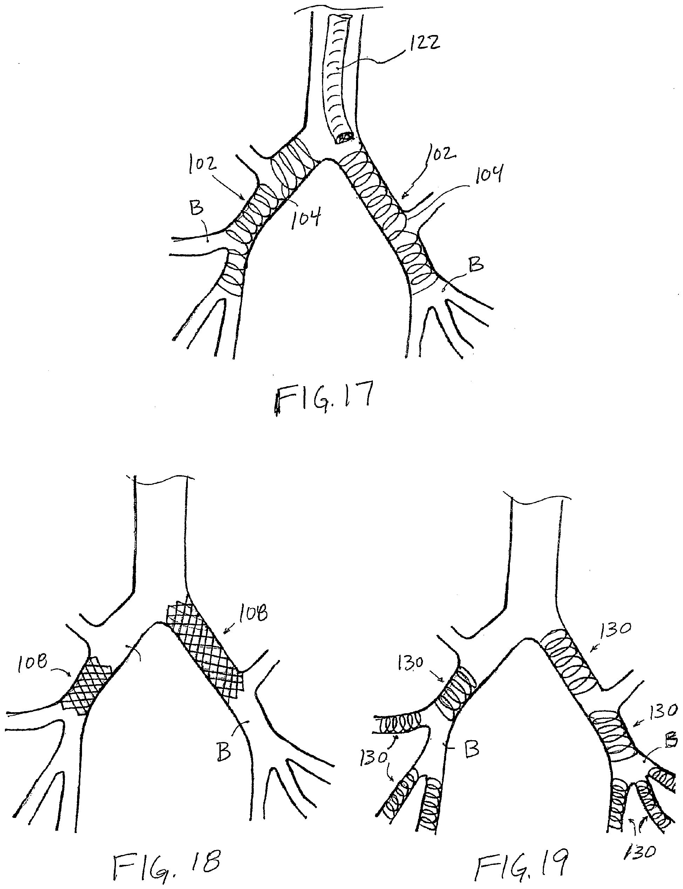

Delivery of the drug-eluting stents preferably is achieved through the working channel of a conventional bronchoscope, either oral or nasal, or through a custom device specifically designed for placing the loaded stents. Referring to FIG. 17, one or more drug-eluting bioabsorbable stents 102 are delivered to target treatment area within a patient's bronchi B through the working channel of bronchoscope 122. Stent 102 is a continuous length of coiled drug-loaded wire 104 that is initially delivered at the distal end of a target bronchial passage. Then, stent 102 is unwound along the target bronchial passage to the desired proximal termination point. Manipulation of the bronchoscope during deployment allows the physician to avoid placing coils across side branches of the bronchial tree. When the proximal termination point is reached, a cutting mechanism is used to cut the implant coil.

Alternatively, as depicted with respect to FIGS. 18 and 19, one or more discrete length drug-eluting bioabsorbable coiled stents 108, 130, may be deployed within the patient's bronchi B. Stents 108 and 130 are formed of wire segments that preferably include alternating layers of drug-loaded and non-drug-loaded materials, such as described with respect to FIG. 16. Stents 108 and 130 preferably are delivered through the working channel of a bronchoscope or through another delivery device. As would be understood by those of skill in the art, stents 108 and 130 may comprise any number of different sizes and configurations.

Deployment of the inventive stents described hereinabove may be accomplished using conventional stent delivery catheters and procedures, via either selfexpansion or balloon expansion of the stents, by heating and setting a final size with a purpose-built delivery system, or any other suitable procedure. Delivery may be achieved one stent at a time, or multiple implants may be placed from a single multi-stent delivery system.

Although preferred illustrative embodiments are described above, it will be evident to one skilled in the art that various changes and modifications may be made without departing from the invention as set out by the appended claims.

## Claims

1. Apparatus for intrabronchial delivery of a neurotoxin to treat lung disease, the apparatus comprising:
a shaft (21) having a proximal end including at least one inlet port, a distal end (23) and a lumen (25) extending between the inlet ports and the distal end;
a neurotoxin applicator (24) disposed on the distal end (23) of the shaft (21) in fluid communication with the lumen (25), the neurotoxin applicator (24) comprising a needle assembly (28) including at least one needle (30) with lumen (31) in fluid communication with lumen (25);
a hilt (36) configured to control the depth of penetration of each of the at least one needle (30) into a bronchial wall;
and
a source of neurotoxin in fluid communication with the lumen (25) and the lumen (31) of the at least one needle (30) of the needle assembly (28).

2. A system comprising the apparatus of claim 1, further comprising a bronchoscope (10) having proximal (12) and distal (13) ends, and a lumen (14) extending therebetween, and wherein the shaft (21) is dimensioned to slidably pass through the lumen (14) of the bronchoscope.

3. The apparatus of claim 1 or 2, wherein the hilt is disposed a pre-selected distance from the distal end of the corresponding needle.

4. The apparatus of claim 3, wherein the needle assembly (28) has a retracted delivery position and a deployed position wherein the at least one needle (30) extends radially outward from the shaft (21) to penetrate the bronchial wall.

5. The apparatus of any one of the preceding claims, wherein the neurotoxin is a serotype of botulinum toxin.

6. The apparatus of claim 5, wherein the serotype of botulinum toxin is selected from the group consisting of serotype A and serotype B.

7. The apparatus of any one of claims 1-5, wherein the neurotoxin is tetanus or a tetanus derivative.

## Patentansprüche

1. Vorrichtung zur intrabronchialen Abgabe eines Neurotoxins zur Behandlung einer Lungenerkrankung, wobei die Vorrichtung Folgendes umfasst:
einen Schaft (21) mit einem proximalen Ende, das mindestens eine Einlassöffnung aufweist, einem distalen Ende (23) und einem Lumen (25), das sich zwischen den Einlassöffnungen und dem distalen Ende erstreckt;
einen Neurotoxin-Applikator (24), der am distalen Ende (23) des Schafts (21) in Fluidverbindung mit dem Lumen (25) angeordnet ist, wobei der Neurotoxin-Applikator (24) eine Nadelanordnung (28) umfasst, die mindestens eine Nadel (30) mit einem Lumen (31) in Fluidverbindung mit dem Lumen (25) aufweist;
einen Griff (36), konfiguriert zum Regeln der Eindringtiefe jeder der mindestens einen Nadel (30) in eine Bronchialwand;
und
eine Neurotoxinquelle in Fluidverbindung mit dem Lumen (25) und dem Lumen (31) der mindestens einen Nadel (30) der Nadelanordnung (28).

2. System, das die Vorrichtung nach Anspruch 1 umfasst und ferner ein Bronchoskop (10) mit einem proximalen (12) und einem distalen (13) Ende und einem sich dazwischen erstreckenden Lumen (14) umfasst, und wobei der Schaft (21) so dimensioniert ist, dass er gleitend durch das Lumen (14) des Bronchoskops geführt werden kann.

3. Vorrichtung nach Anspruch 1 oder 2, wobei der Griff in einem vorgewählten Abstand vom distalen Ende der entsprechenden Nadel angeordnet ist.

4. Vorrichtung nach Anspruch 3, wobei die Nadelanordnung (28) eine eingefahrene Abgabeposition und eine ausgefahrene Position aufweist, in der sich die mindestens eine Nadel (30) vom Schaft (21) radial nach außen erstreckt, um die Bronchialwand zu durchdringen.

5. Vorrichtung nach einem der vorherigen Ansprüche, wobei das Neurotoxin ein Serotyp von Botulinumtoxin ist.

6. Vorrichtung nach Anspruch 5, wobei der Serotyp des Botulinumtoxins ausgewählt ist aus der Gruppe bestehend aus Serotyp A und Serotyp B.

7. Vorrichtung nach einem der Ansprüche 1-5, wobei das Neurotoxin Tetanus oder ein Tetanusderivat ist.

## Revendications

1. Appareil destiné à la délivrance intrabronchique d'une neurotoxine pour traiter une maladie pulmonaire, l'appareil comprenant :
un arbre (21) ayant une extrémité proximale incluant au moins un orifice d'entrée, une extrémité distale (23) et une lumière (25) s'étendant entre les orifices d'entrée et l'extrémité distale ;
un applicateur de neurotoxine (24) disposé sur l'extrémité distale (23) de l'arbre (21) en communication fluidique avec la lumière (25), l'applicateur de neurotoxine (24) comprenant un ensemble d'aiguille (28) incluant au moins une aiguille (30) avec une lumière (31) en communication fluidique avec la lumière (25) ;
une garde (36) configurée pour contrôler la profondeur de pénétration de chacune de l'au moins une aiguille (30) jusque dans une paroi bronchique ;
et
une source de neurotoxine en communication fluidique avec la lumière (25) et la lumière (31) de l'au moins une aiguille (30) de l'ensemble d'aiguille (28).

2. Système comprenant l'appareil selon la revendication 1, comprenant en outre un bronchoscope (10) ayant des extrémités proximale (12) et distale (13), et une lumière (14) s'étendant entre celles-ci, et dans lequel l'arbre (21) est dimensionné pour passer de manière coulissante à travers la lumière (14) du bronchoscope.

3. Appareil selon la revendication 1 ou 2, dans lequel la garde est disposée à une distance présélectionnée de l'extrémité distale de l'aiguille correspondante.

4. Appareil selon la revendication 3, dans lequel l'ensemble d'aiguille (28) a une position de délivrance rétractée et une position déployée dans lequel l'au moins une aiguille (30) s'étend de manière radiale vers l'extérieur depuis l'arbre (21) pour pénétrer dans la paroi bronchiale.

5. Appareil selon l'une quelconque des revendications précédentes, dans lequel la neurotoxine est un sérotype de toxine botulinique.

6. Appareil selon la revendication 5, dans lequel le sérotype de toxine botulinique est choisi parmi le groupe constitué par le sérotype A et le sérotype B.

7. Appareil selon l'une quelconque des revendications 1 à 5, dans lequel la neurotoxine est le tétanos ou un dérivé du tétanos.
